# EUROPÄISCHE PATENTANMELDUNG

(11) **EP 2 025 287 A1**
(43) Veröffentlichungstag der Anmeldung: **18.02.2009**
(21) Anmeldenummer: 07114414.1
(22) Anmeldetag: 16.08.2007
(51) Int. Cl.: A61B 5/151

(54) **Diagnostisches Einmalteil und Verfahren zu seiner Herstellung**

(71) Anmelder: F.HOFFMANN-LA ROCHE AG, 4070 Basel (CH); Roche Diagnostics GmbH, 68305 Mannheim (DE)
(72) Erfinder: Fürst, Otto, Dr., 68519 Viernheim (DE); Haar, Hans-Peter, Dr., 69168 Wiesloch (DE)
(74) Vertreter: Pfiz, Thomas

(57) **Zusammenfassung**

Die Erfindung betrifft ein diagnostisches Einmalteil mit einem Stechelement (14), das zum Einstechen in die Haut (26) ausgebildet ist und einen Sammelkanal (16) zum Aufnehmen von Körperflüssigkeit aufweist, und einem Nachweiselement (18), das eine mit Reagenzien für den Nachweis eines Analyten in der Körperflüssigkeit versehene Testschicht (42) aufweist und in dem Sammelkanal (16) angeordnet ist. Um eine verbesserte Stabilität und Biokompatibilität sicherzustellen, wird vorgeschlagen, dass das Nachweiselement (18) mit einer auf der Testschicht (42) aufgebrachten, die Reagenzien abdeckenden Versiegelungsschicht (20) versehen ist.

## Beschreibung

Die Erfindung betrifft ein diagnostisches Einmalteil mit einem Stechelement, das zum Einstechen in die Haut ausgebildet ist und einen vorzugsweise seitlich offenen Sammelkanal zum Aufnehmen von Körperflüssigkeit aufweist, und einem Nachweiselement, das eine mit Reagenzien, insbesondere Enzymen für den Nachweis eines Analyten in der Körperflüssigkeit versehene Testschicht aufweist und in dem Sammelkanal angeordnet ist. Die Erfindung betrifft weiter ein Herstellungsverfahren für ein solches disposibles Teil.

Für Blutzucker-Selbstkontrollen bei Diabetikern ist es wünschenswert, der betroffenen Person möglichst wenige Handhabungsschritte aufzuerlegen und zugleich eine schmerzarme und zuverlässige Messung sicherzustellen. Dabei werden aus hygienischen Gründen Einmalartikel für einen Hauteinstich eingesetzt. Bei neueren Konzepten, wie sie beispielsweise in der WO 2007/045412 ausgeführt sind, wird ein vereinfachter Probentransfer durch Integration des Nachweiselements in das disposible Teil angestrebt, um auch mit geringsten Probenmengen einen robusten Messvorgang zu gewährleisten.

Ausgehend hiervon liegt der Erfindung die Aufgabe zugrunde, die im Stand der Technik bekannten Probensammler und Verfahren zu deren Herstellung weiter zu verbessern und so zu gestalten, dass eine unproblematische Probenaufnahme und zuverlässige Analyterfassung ermöglicht wird.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, Einmalteile einzusetzen, bei denen die Probe in einem kleinen Volumen möglichst nahe am Stechorgan biokompatibel und zuverlässig analysiert werden kann. Dementsprechend wird erfindungsgemäß vorgeschlagen, dass das in dem Sammelkanal integrierte Nachweiselement mit einer auf der Testschicht aufgebrachten, die Reagenzien abdeckenden Versiegelungsschicht versehen ist. Dadurch kann die Integrität der Testschicht auch während des Zusammenbaus und der Lagerzeit verbessert werden, während zugleich gewährleistet ist, dass die Testchemie nicht in direkten Hautkontakt gelangt. Ebenso kann dadurch sichergestellt werden, dass sich keine Feststoffpartikel von der mit enzymbehafteten Trockensubstanzen versehenen Testschicht lösen und im Körper allergische Reaktionen auslösen. Dies ist angesichts der hohen Zahl und täglich erforderlichen Wiederholung der Anwendung von besonderer Bedeutung. Durch die Versiegelung ist es auch möglich, das Nachweiselement weiter nach distal in die Stechpartie zu verlagern, so dass auch das Sammelvolumen weiter reduziert werden kann.

Wichtig ist dabei, dass die Messdauer nicht für den Anwender unzumutbar verlängert wird. Eine bevorzugte Ausführung sieht daher vor, dass die Versiegelungsschicht bei Befüllung des Sammelkanals durch die Körperflüssigkeit löslich ist, so dass der Analyt in Kontakt mit den Reagenzien kommt. Hierbei ist es vorteilhaft, wenn die Lösezeit zum Auflösen der Versiegelungsschicht in der Körperflüssigkeit weniger als 10 s, vorzugsweise weniger als 2 s beträgt.

Um ein rasches Auflösen zu unterstützen, ist es günstig, wenn die Versiegelungsschicht als Flüssigkeitsfilm ausgebildet ist.

Zur weiteren Verbesserung des Schutzes gegen Ablösen von Partikeln ist es von besonderem Vorteil, wenn die Versiegelungsschicht einen die Testschicht begrenzenden Rand des Nachweiselements umschließt.

In baulich vorteilhafter Ausgestaltung weist das Nachweiselement einen aus einem Flachmaterial gebildeten Träger auf. Vorteilhaft ist es auch, wenn das Stechelement aus Metall, insbesondere Stahl besteht.

Um die Testeigenschaften möglichst nicht zu beeinflussen und die Schichtanpassung zu optimieren, ist es vorteilhaft, wenn die Testschicht mit einer auch in der Versiegelungsschicht enthaltenen Substanz versehen ist.

Für die Beaufschlagung mit der beim Einstich gewonnenen Körperflüssigkeit ist es von Vorteil, wenn das Nachweiselement in einen Endabschnitt des Sammelkanals fest eingesetzt ist, wobei die Testschicht in Stechrichtung des Stechelements ausgerichtet ist.

Ein besonderer Aspekt der Erfindung liegt darin, dass die Versiegelungsschicht aus nicht-ionischen Tensiden gebildet ist. In diesem Zusammenhang ist es auch denkbar, dass nur eine Partie des Stechelements mit einer aus nicht-ionischen Tensiden gebildeten Beschichtung versehen ist, vor allem um die Hydrophilie zu verbessern und eine für die Blutaufnahme optimierte Oberfläche zu schaffen. Hierbei ist es möglich, dass die abriebfeste Versiegelungsschicht eine in die Haut eindringende Partie des Stechelements bedeckt.

Besonders bevorzugt kommen Polysorbate, vorzugsweise Polysorbat 20 und/oder Polysorbat 80 zum Einsatz. Denkbar ist es auch, dass die nicht-ionischen Tenside Poloxamer, vorzugsweise Poloxamer 188 enthalten.

Eine weitere vorteilhafte Ausgestaltung sieht vor, dass die nicht-ionischen Tenside mindestens eine Substanz ausgewählt aus der Gruppe Fettalkohol-Polyglycol-Ether, Glucamide, Fettalkohol-Ethoxylate, Alkyl-Poly-Glycoside, Saccharose-Fettsäure-Ester enthalten.

Gegenstand der Erfindung ist auch ein Magazin zur Aufnahme einer Mehrzahl von erfindungsgemäßen diagnostischen Einmalteilen für den Einsatz in einem Handgerät insbesondere zur Blutzuckerbestimmung, sowie ein System für die Untersuchung einer Körperflüssigkeit, insbesondere als Handgerät zur Blutzuckerbestimmung mit mindestens einem darin angeordneten oder einsetzbaren erfindungsgemäßen diagnostischen Einmalteil.

In verfahrensmäßiger Hinsicht wird die eingangs genannte Aufgabe dadurch gelöst, dass das Stechelement und/oder das Nachweiselement mit nicht-ionischen Tensiden als Beschichtungsmaterial zumindest partiell beschichtet wird. Dies lässt sich dadurch günstig bewerkstelligen, dass das Beschichtungsmaterial in einem zumindest überwiegend wasserfreien Lösemittel, vorzugsweise Ethanol auf die zu beschichtende Oberfläche aufgebracht wird. Das Lösemittel kann sodann nach dem Beschichten durch einen Gasstrom, Unterdruck und/oder Erwärmen entfernt werden.

Vorteilhafterweise wird das Beschichtungsmaterial durch Tauch-, Sprüh- oder Kontaktbeschichten auf die zu beschichtende Oberfläche aufgebracht. Hierbei kann es von Vorteil sein, wenn die Viskosität des Beschichtungsmaterials durch Zusatzkomponenten eingestellt wird.

Eine weitere Verbesserung sieht vor, dass das Nachweiselement aus einem Flachmaterial zugeschnitten wird, und dass das Beschichtungsmaterial auf das zugeschnittene Nachweiselement vor oder nach dem Einsetzen in das Stechelement aufgebracht wird.

Herstellungstechnisch ist es auch von Vorteil, wenn das Nachweiselement nach dem Beschichten auf einen Träger, insbesondere stirnseitig auf einen Lichtleiter aufgesetzt wird, und dass anschließend die Montageeinheit aus Träger und Nachweiselement mit dem Stechelement zusammengesetzt wird.

Im Folgenden wird die Erfindung anhand eines in der Zeichnung schematisch dargestellten Ausführungsbeispiels näher erläutert. Es zeigen:
- Fig. 1: ein Blutzuckermessgerät mit einem darin eingesetzten Micro-sampler als diagnostisches Einmalteil in einer schaubildlichen Darstellung;
- Fig. 2: den Microsampler in der Draufsicht; einer abgebrochenen perspektivischen Darstellung;
- Fig. 3: den Microsampler in einer abgebrochenen Seitenansicht;
- Fig. 4: einen vergrößerten Ausschnitt aus Fig. 2.

Die in der Zeichnung dargestellten diagnostischen Einmalteile 10 lassen sich als mikrofluidische Probensammler bzw. Microsampler zur Blutzuckerbestimmung in einem dafür ausgebildeten Handgerät 12 einsetzen, wobei mit einer minimalen Probenmenge in dem Einmalteil ein Glucosenachweis durchführbar ist. Zu diesem Zweck umfassen die Microsampler 10 ein Stechelement 14 mit einem schlitzförmigen, beidseitig offenen Sammelkanal 16 und ein darin angeordnetes Nachweiselement 18 für eine optische oder elektrochemische Messung direkt in dem Sammelkanal 16, wobei eine das Nachweiselement 18 und gegebenenfalls auch das Stechelement 14 mit einer speziellen Beschichtung 20 aus nicht-ionischen Tensiden versehen ist. Ein Halter 22 für das Stechelement und das Nachweiselement ermöglicht eine Kopplung mit einem Stechantrieb 24 für einen Einstich in die Haut 26 beispielsweise eines Fingers eines Benutzers.

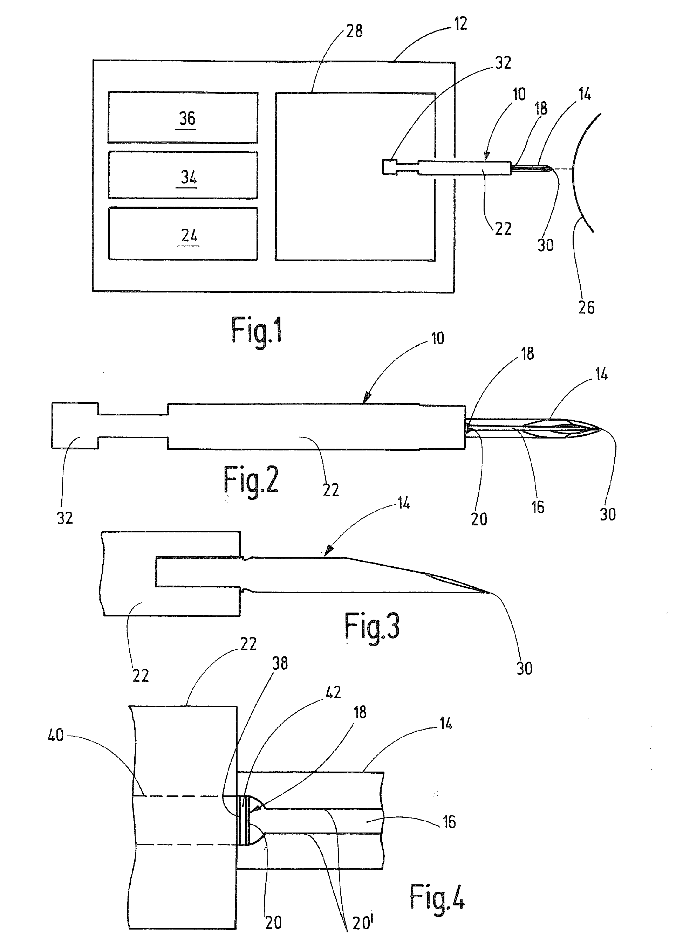

Wie in Fig. 1 veranschaulicht, lassen sich die Microsampler 10 in einem in das Gerät 12 einsetzbaren Magazin 28 sukzessive in eine aktive Gebrauchsposition bringen. Das aktive Stechelement 14 weist dabei mit seiner Spitze 30 in distaler Richtung zu dem Körperteil 26 hin, während ein Kopplungsende 32 des Halters 22 für eine Antriebs- und Signalkopplung mit dem Stechantrieb 24 gekoppelt ist. Die während des Hauteinstichs in dem Sammelkanal 16 aufgenommene Körperflüssigkeit (Blut bzw. Gewebeflüssigkeit) kann über das Nachweiselement 18 direkt photometrisch oder elektrochemisch untersucht werden, wobei die Signalauswertung in einer geräteseitigen Auswerteeinheit 34 erfolgt. Dabei ist es auch möglich, das Messergebnis auf einer Anzeige 36 für den Benutzer anzuzeigen, so dass eine Blutzuckerkontrolle ohne aufwändige Handhabungsschritte vor Ort möglich ist.

Wie aus Fig. 2 ersichtlich, weist das schaftförmig-langgestreckte Stechelement 14 einen quer durchgehenden Längsschlitz als Sammelkanal 16 auf. Dieser ermöglicht gegebenenfalls durch Kapillarwirkung den Transfer einer mikroskopischen Flüssigkeitsmenge auf das in Richtung der Spitze 30 ausgerichtete Nachweiselement 18. Durch die langgestreckte beidseitige Schlitzöffnung wird eine effektive Flüssigkeitsaufnahme ohne die Gefahr einer Verstopfung durch Zellbestandteile gewährleistet. Für eine möglichst schonende und schmerzarme Blutentnahme ist es vorgesehen, dass das Volumen des Sammelkanals 16 lediglich einige 10 Nanoliter beträgt.

In der Seitenansicht gemäß Fig. 3 ist erkennbar, dass das Stechelement 14 an seinem proximalen Ende klammerartig auf den mit seitlichen Nuten versehenen Halter 22 aufgesteckt ist, so dass das stirnseitig auf dem Halter 22 aufgebrachte Nachweiselement 18 in den Sammelkanal 16 eingreift. Der distale Abschnitt des aus Stahl gebildeten Stechelements 14 ist schräg zu der Spitze 30 hin angeschliffen, um den Einstich in die Haut durch einen verringerten Querschnitt zu erleichtern.

Fig. 4 zeigt das aus mehreren Lagen aufgebaute Nachweiselement 18 in einem vergrößerten Ausschnitt. Als Basis ist ein transparenter Träger 38 vorgesehen, der auf einem den Halter 22 durchsetzende Lichtleiteranordnung 40 sitzt. Auf dem Träger 38 befindet sich eine Testschicht 42, die mit Reagenzien für einen Glucosenachweis in der in dem Sammelkanal 16 gewonnenen Blutflüssigkeit und ggf. weiteren Hilfsstoffen versehen ist. Die Reagenzien können durch ein an sich bekanntes enzymatisches System gebildet sein, welches mit Blutglucose unter Farbänderung irreversibel reagiert, sich aber nicht in der Blutflüssigkeit löst. Durch streuende Partikel innerhalb des Chemiesystems wird unter Rückstreuung des über die Lichtleiteranordnung 40 eingestrahlten Messlichts eine geräteseitige optische Detektion ermöglicht.

Die Versiegelungsschicht 20 überdeckt die Testschicht 42 und versiegelt damit die Reagenzien, so dass zunächst eine gute Lagerstabilität erreicht und ein Ablösen von Reagenzpartikeln von der trockengelagerten Testfläche vermieden wird. Durch die Versiegelung ergibt sich auch ein wichtiger Gebrauchsvorteil dahingehend, dass ein direkter Hautkontakt mit der Testfläche oder davon abgelösten Substanzen beim Einstich verhindert wird. Zweckmäßig umschließt die Versiegelungsschicht 20 auch die Seitenränder der Testfläche 42, um ein Ablösen von Teilchen zuverlässig auszuschließen. Dies ist besonders dann wichtig, wenn das Nachweiselement 18 durch Zuschneiden aus einem großflächigen beschichteten Folienmaterial gebildet ist.

Um den Nachweis des Analyten nicht wesentlich zu behindern, ist die Versiegelungsschicht 20 bei Beaufschlagung mit der in den Sammelkanal 16 einströmenden Blutflüssigkeit löslich. Die erforderliche Zeit für ein hinreichendes Auflösen, d.h. die Zeit bis zum Erhalt eines auswertbaren Messsignals sollte weniger als 2 s betragen, um den Benutzungskomfort nicht einzuschränken. Entsprechend sollte die Versiegelungsschicht 20 eine hohe Hydrophilie besitzen, wobei eine Ausbildung als Flüssigkeitsfilm vorteilhaft ist.

Es versteht sich, dass die Versiegelungsschicht 20 biokompatibel sein sollte, um nicht selbst bei einem Hautkontakt nachteilige Reaktionen auszulösen. Dies lässt sich besonders zuverlässig dadurch erreichen, dass pharmakologisch und ggf. auch lebensmittelchemisch zugelassene unbedenkliche Substanzen als Beschichtungsmaterial eingesetzt werden. Solche Substanzen sollen aber im Rahmen der pharmakologischen Zulassung keine eigenständigen pharmakologischen funktionalen Eigenschaften haben.

Schließlich darf das Beschichtungsmaterial die Testreagenzien und den Testablauf und damit das Ergebnis nicht beeinflussen, gleichgültig ob ein Kontakt mit der Testfläche bei der Herstellung, Lagerung oder erst bei der Probenmessung stattfindet. Idealerweise sollte eine bestehende Testchemie nicht nur hinsichtlich des Beschichtungsmaterials verträglich sein, sondern als Testfläche 42 dieses Material zweckmäßig auch selbst enthalten. So kann die Testfläche 42 als Benetzungsmittel, welches ohnehin benötigt wird, hierfür diejenige Substanz enthalten, welche auch als Versiegelungsmaterial eingesetzt wird. Damit lassen sich bedenklichen Konzentrationsgradienten oder nachteilige Veränderungen im Gesamtsystem vermeiden.

Eine Beschichtung 20' des Stechelements 14 mit kann sich ebenfalls als vorteilhaft erweisen, um eine hydrophile Oberfläche für die Aufnahme der Körperflüssigkeit insbesondere im Bereich des Sammelkanals 16 zu schaffen. Denkbar ist es auch, durch eine Beschichtung im Bereich der Spitze 30 die Reibung beim Einstich und damit den Stechschmerz zu verringern.

In diesem Zusammenhang haben sich nicht-ionische Tenside und vor allem Polysorbate als besonders geeignetes Beschichtungsmaterial erwiesen, um die vorgenannten Anforderungen zu erfüllen und eine Massenherstellung von Einmalartikeln praktischerweise zu ermöglichen. Besonders bevorzugt wird Polysorbat 20 (PS 20) eingesetzt, welches die folgenden Vorteile aufweist:
- PS 20 wird in Medikamenten (Injektions-Lösungen) verwendet und ist auch in Nahrungsmitteln zu finden.
- PS 20 lässt sich lange lagern und wirkt auch noch lange nach Verfallsdatum.
- PS 20 ist hochmolekular, diffundiert also kaum durch Körpergewebe;
- PS 20 ist bei Raumtemperatur dick-flüssig und löst sich gut im Lösemittel Ethanol (und nicht nur in Wasser).
- PS 20 im Lösemittel Ethanol ist steril, verkeimt nicht und entkeimt benetzte Bereiche durch das Lösemittel.
- PS 20 lässt sich in ethanolischer Lösung leicht auftragen und dringt wegen der geringen Lösemittel-Viskosität in kapillare Strukturen rasch und gut ein.
- PS 20, aufgetragen aus Ethanol, lässt sich rasch und zuverlässig von diesem befreien (z.B. durch Luftstrom, Unterdruck)
- PS 20 bildet kein Kristallgitter und löst sich folglich bei Kontakt mit dem Wasser der Probe (Blut) rasch auf, da keine hohe Lösungsenthalpie zur Überwindung von Gitterkräften erforderlich ist. Eine rasche Interaktion mit der Probe führt auch zu rascher Benetzung.
- PS 20, kriecht auch (mengenabhängig) nach dem Trocknen in feinste Strukturen. Die Hydrophilie und Benetzungsgeschwindigkeit scheint nach einiger Zeit des Lagerns noch besser - und nicht schlechter - zu werden.
- PS 20 zeigte in ersten Experimenten keine erkennbare Beeinflussung der Nachweisreaktion.
- PS 20 sollte eine geringe, aber nicht funktionale Gerinnungshemmung bewirken (und zwar nur in dem Sammelkanal, während außerhalb davon die Verdünnung zu hoch ist).

Die Konsistenz bzw. Viskosität von PS 20 lässt sich ggf. durch Zusatz anderer Komponenten optimieren. Beispielsweise könnte eine Zugabe von Poloxamer 188 (fest) die Kriechfähigkeit des PS 20 geeignet modifizieren. Weiterhin hat sich auch Polysorbat 80 als geeignetes Beschichtungsmaterial herausgestellt.

Weitere denkbare Stoffklassen, die zwar körperfremd, jedoch in der Lebensmittelchemie und Biochemie bzw. Molekularbiologie zum Einsatz kommen, sind folgende Detergenzien:
- AEOs: Fettalkohol-Polyglycol-Ether

   CH3-(CH2)16-CH2-(OCH2CH2)n-OH n=1-20
- Glucamide, abgeleitet von Fettsäuren und Glucose

   R-CO-NH-CH2-(CH2)4-CH2-OH R=C11H23
- Fettalkohol-Ethoxylate
- APGs Alkyl-Poly-Glycoside

   Polyglycosil-O-(CH2)n-CH3 n=z.B. 12
- Saccharose-Fettsäure-Ester

   (Sacchh.:Glucose-C6-)-O-CO-(CH2)n-CH3 n=10-16

Selbstverständlich sind auch Kombinationen dieser Substanzen untereinander oder mit anderen Substanzen denkbar.

Bei der Herstellung von diagnostischen Einmalartikeln 10 ist es also vorteilhaft, wenn das Stechelement 14 und/oder das Nachweiselement 18 mit nicht-ionischen Tensiden als Beschichtungsmaterial zumindest partiell beschichtet wird. Dies kann dadurch erfolgen, dass das Beschichtungsmaterial durch Tauch-, Sprüh- oder Kontaktbeschichten auf die zu beschichtende Oberfläche aufgebracht wird. Zweckmäßig wird das Beschichtungsmaterial in Ethanol als Lösemittel auf die zu beschichtende Oberfläche aufgebracht wird, und anschließend das Lösemittel entfernt, beispielsweise durch einen Gasstrom, Unterdruck und/oder Erwärmen.

Das Nachweiselement 18 kann als zugeschnittenes Flachmaterialstück nach dem Beschichten auf dem Träger 22, d.h. stirnseitig auf dem Lichtleiter 40 fixiert werden, wobei anschließend die Montageeinheit aus Träger 22 und Nachweiselement 18 mit dem Stechelement 14 zusammengefügt wird. Weitere Einzelheiten hierzu ergeben sich aus der Patentanmeldung PCT/US07/65918, worauf in diesem Zusammenhang ausdrücklich Bezug genommen wird.

## Patentansprüche

1. Diagnostisches Einmalteil mit einem Stechelement (14), das zum Einstechen in die Haut (26) ausgebildet ist und einen vorzugsweise seitlich offenen Sammelkanal (16) zum Aufnehmen von Körperflüssigkeit aufweist, und einem Nachweiselement (18), das eine mit Reagenzien, insbesondere Enzymen für den Nachweis eines Analyten in der Körperflüssigkeit versehene Testschicht (42) aufweist und in dem Sammelkanal (16) angeordnet ist, **dadurch gekennzeichnet, dass** das Nachweiselement (18) mit einer auf der Testschicht (42) aufgebrachten, die Reagenzien abdeckenden Versiegelungsschicht (20) versehen ist.

2. Diagnostisches Einmalteil nach Anspruch 1, **dadurch gekennzeichnet, dass** die Versiegelungsschicht (20) bei Befüllung des Sammelkanals (16) durch die Körperflüssigkeit löslich ist, so dass der Analyt in Kontakt mit den Reagenzien kommt.

3. Diagnostisches Einmalteil nach Anspruch 1 oder 2, **dadurch gekennzeichnet, dass** die Lösezeit zum Auflösen der Versiegelungsschicht (20) in der Körperflüssigkeit weniger als 10 s, vorzugsweise weniger als 2 s beträgt.

4. Diagnostisches Einmalteil nach einem der Ansprüche 1 bis 3, **dadurch gekennzeichnet, dass** die Versiegelungsschicht (20) als Flüssigkeitsfilm ausgebildet ist.

5. Diagnostisches Einmalteil nach einem der Ansprüche 1 bis 4, **dadurch gekennzeichnet, dass** die Versiegelungsschicht (20) einen die Testschicht (42) begrenzenden Rand des Nachweiselements (18) umschließt.

6. Diagnostisches Einmalteil nach einem der Ansprüche 1 bis 5, **dadurch gekennzeichnet, dass** das Nachweiselement (18) einen aus einem Flachmaterial gebildeten Träger (38) aufweist.

7. Diagnostisches Einmalteil nach einem der Ansprüche 1 bis 6, **dadurch gekennzeichnet, dass** das Stechelement (14) aus Metall, insbesondere Stahl besteht.

8. Diagnostisches Einmalteil nach einem der Ansprüche 1 bis 7, **dadurch gekennzeichnet, dass** die Testschicht (42) mit einer auch in der Versiegelungsschicht (20) enthaltenen Substanz versehen ist.

9. Diagnostisches Einmalteil nach einem der Ansprüche 1 bis 8, **dadurch gekennzeichnet, dass** das Nachweiselement (18) in einen Endabschnitt des Sammelkanals (16) fest eingesetzt ist, wobei die Testschicht (42) in Stechrichtung des Stechelements (14) ausgerichtet ist.

10. Diagnostisches Einmalteil nach einem der Ansprüche 1 bis 9, **dadurch gekennzeichnet, dass** die Versiegelungsschicht (20) aus nicht-ionischen Tensiden gebildet ist.

11. Diagnostisches Einmalteil mit einem Stechelement (14), das zum Einstechen in die Haut (26) ausgebildet ist und einen vorzugsweise seitlich offenen Sammelkanal (16) zum Aufnehmen von Körperflüssigkeit aufweist, und gegebenenfalls einem Nachweiselement (18), das eine mit Reagenzien, insbesondere Enzymen für den Nachweis eines Analyten in der Körperflüssigkeit versehene Testschicht (42) aufweist, **dadurch gekennzeichnet, dass** das Stechelement (14) und/oder das Nachweiselement (18) zumindest partiell mit einer aus nicht-ionischen Tensiden gebildeten Versiegelungsschicht (20) versehen ist.

12. Diagnostisches Einmalteil nach Anspruch 10 oder 11, **dadurch gekennzeichnet, dass** die nicht-ionischen Tenside ein Polysorbat, vorzugsweise Polysorbat 20 und/oder Polysorbat 80 enthalten.

13. Diagnostisches Einmalteil nach einem der Ansprüche 10 bis 12, **dadurch gekennzeichnet, dass** die nicht-ionischen Tenside Poloxamer, vorzugsweise Poloxamer 188 enthalten.

14. Diagnostisches Einmalteil nach einem der Ansprüche 10 bis 13, **dadurch gekennzeichnet, dass** die nicht-ionischen Tenside mindestens eine Substanz ausgewählt aus der Gruppe Fettalkohol-Polyglycol-Ether, Glucamide, Fettalkohol-Ethoxylate, Alkyl-Poly-Glycoside, Saccharose-Fettsäure-Ester enthalten.

15. Diagnostisches Einmalteil nach einem der Ansprüche 1 bis 14, **dadurch gekennzeichnet, dass** die Versiegelungsschicht (20) eine in die Haut (26) eindringende Partie des Stechelements (14) bedeckt.

16. Diagnostisches Einmalteil nach einem der Ansprüche 1 bis 15, **dadurch gekennzeichnet, dass** die Versiegelungsschicht (20) eine hydrophile Oberfläche für die Aufnahme von Körperflüssigkeit beim Einstechen in die Haut (26) bildet.

17. Magazin (28) zur Aufnahme einer Mehrzahl von diagnostischen Einmalteilen(10) nach einem der vorhergehenden Ansprüche für den Einsatz in einem Handgerät (12) insbesondere zur Blutzuckerbestimmung.

18. System für die Untersuchung einer Körperflüssigkeit, insbesondere als Handgerät zur Blutzuckerbestimmung mit mindestens einem darin angeordneten oder einsetzbaren diagnostischen Einmalteil (10) nach einem der Ansprüche 1 bis 16.

19. Verfahren zur Herstellung eines diagnostischen Einmalteils mit einem Stechelement (14), das zum Einstechen in die Haut (26) ausgebildet ist und einen vorzugsweise seitlich offenen Sammelkanal (16) zum Aufnehmen von Körperflüssigkeit aufweist, und gegebenenfalls einem Nachweiselement (18), das eine für den Nachweis eines Analyten in der Körperflüssigkeit ausgebildete Testschicht (42) aufweist, **dadurch gekennzeichnet, dass** das Stechelement (14) und/oder das Nachweiselement (18) mit nicht-ionischen Tensiden als Beschichtungsmaterial zumindest partiell beschichtet wird.

20. Verfahren nach Anspruch 19, **dadurch gekennzeichnet, dass** das Beschichtungsmaterial in einem zumindest überwiegend wasserfreien Lösemittel, vorzugsweise Ethanol auf die zu beschichtende Oberfläche aufgebracht wird.

21. Verfahren nach Anspruch 19 oder 20, **dadurch gekennzeichnet, dass** das Lösemittel nach dem Beschichten durch einen Gasstrom oder Unterdruck oder Erwärmen entfernt wird.

22. Verfahren nach einem der Ansprüche 19 bis 21, **dadurch gekennzeichnet, dass** das Beschichtungsmaterial durch Tauch-, Sprüh- oder Kontaktbeschichten auf die zu beschichtende Oberfläche aufgebracht wird.

23. Verfahren nach einem der Ansprüche 19 bis 22, **dadurch gekennzeichnet, dass** die Viskosität des Beschichtungsmaterials durch Zusatzkomponenten eingestellt wird.

24. Verfahren nach einem der Ansprüche 19 bis 23, **dadurch gekennzeichnet, dass** das Nachweiselement (18) aus einem Flachmaterial zugeschnitten wird, und dass das Beschichtungsmaterial auf das zugeschnittene Nachweiselement (18) vor oder nach dem Einsetzen in das Stechelement (14) aufgebracht wird.

25. Verfahren nach einem der Ansprüche 19 bis 24, **dadurch gekennzeichnet, dass** das Nachweiselement (18) nach dem Beschichten auf einen Träger, insbesondere stirnseitig auf einen Lichtleiter aufgesetzt wird, und dass anschließend die Montageeinheit aus Träger und Nachweiselement (18) mit dem Stechelement (14) zusammengesetzt wird.
